# EUROPEAN PATENT APPLICATION

(11) **EP 4 530 288 A1**
(43) Date of publication of application: **02.04.2025**
(21) Application number: 23834743.9
(22) Date of filing: 30.06.2023
(51) Int. Cl.: C07D 487/04, C07D 231/16, A61K 31/519, A61P 29/00, A61P 35/00

(54) **COMPOUND CONTAINING GEM-DIFLUORO GROUP, PREPARATION METHOD THEREFOR AND USE THEREOF**

(30) Priority: 05.07.2022 CN 202210784219
(71) Applicant: CGeneTech (Suzhou, China) Co., Ltd., Suzhou, Jiangsu 215123 (CN)
(72) Inventor: WANG, Tong, Suzhou, Jiangsu 215123 (CN); HAO, Yan, Suzhou, Jiangsu 215123 (CN); XU, Yudong, Suzhou, Jiangsu 215123 (CN); ZHANG, Fuzhi, Suzhou, Jiangsu 215123 (CN); YU, Qiang, Suzhou, Jiangsu 215123 (CN); DING, Juping, Suzhou, Jiangsu 215123 (CN); CHEN, Qingfei, Suzhou, Jiangsu 215123 (CN); LU, Qin, Suzhou, Jiangsu 215123 (CN)
(74) Representative: LLR
(86) International application number: PCT/CN2023/104417
(87) International publication number: WO 2024/007976

(57) **Abstract**

The present application provide a compound represented by formula (I), a chiral enantiomer thereof or a pharmaceutically acceptable salt thereof, and a preparation method therefor, an intermediate for preparing the compound represented by formula (I), the chiral enantiomer thereof or the pharmaceutically acceptable salt thereof, and a preparation method therefor, a pharmaceutical composition comprising the compound represented by formula (I), the chiral enantiomer thereof or the pharmaceutically acceptable salt thereof, and pharmaceutical use of the compound represented by formula (I), the chiral enantiomer thereof or the pharmaceutically acceptable salt thereof or the pharmaceutical composition comprising the same. The compounds of the present application exhibit good JAK2 kinase inhibitory activity and high selectivity for JAK2 kinase. In addition, the compounds of the present application have significant pharmacokinetic advantages, provide more options for the prevention and/or treatment of diseases associated abnormal JAK signaling pathways, and have a good application prospect in clinical practice.

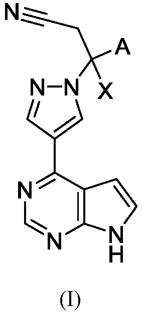

## Description

### Cross-reference to Related Application

The present application claims the priority of Chinese Patent Application No. 202210784219.X, filed on July 5, 2022, and entitled "Compounds containing a gem-difluoride group and preparation method and use thereof', the entire contents of which are incorporated herein by reference.

### Technical Field

The present application relates to the field of pharmaceutical chemistry, in particular to compounds containing a gem-difluoride group and preparation method and use thereof.

### Background Art

Janus kinase (JAK) is a class of tyrosine kinases. The JAK family includes four members: JAK1, JAK2, JAK3, and TYK2. JAKs play an important role in the signal transduction of various cytokines.

Signal transducer and activator of transcription (STAT) is a group of cytoplasmic proteins that bind to DNA in the regulatory regions of target genes. As the downstream substrates of JAKs, STATs can be activated by tyrosine phosphorylation under the stimulation of external signals, and then translocate into the nucleus to regulate gene transcription.

Many abnormal immune responses, such as allergy, asthma, allograft rejection, autoimmune diseases such as rheumatoid arthritis and multiple sclerosis, as well as hematological malignancies such as myelodysplasia, leukemia, and lymphoma, are related to the dysregulation of the JAK/STAT signaling pathway.

JAK protein kinase inhibitors, particularly JAK3 protein kinase inhibitors, can prevent the activation of T cells, prevent graft rejection after transplantation, and have therapeutic effects on autoimmune diseases such as rheumatoid arthritis and multiple sclerosis. In addition, myeloproliferative neoplastic diseases, including essential thrombocythemia, polycythemia vera, and primary myelofibrosis, have been found to be associated with abnormal activity due to mutations in the JAK2 kinase. Therefore, the development of JAK2 protein kinase inhibitors has great medical value and market potential for the treatment of myeloproliferative tumors. Ruxolitinib is a selective JAK1/2 kinase inhibitor, which has been approved by FDA and marketed as Jakafi as early as November 16, 2011. It is the first drug approved for the treatment of primary myelofibrosis (PMF) in the United States. However, it lacks high selectivity for the JAK2 kinase and has a short half-life and low exposure.

Therefore, there remains a need to develop new compounds with higher selectivity for the JAK2 kinase and improved pharmacokinetics.

### Summary of the Invention

The present application aims to provide a compound represented by formula (I), a chiral enantiomer thereof, or a pharmaceutically acceptable salt thereof, and a preparation method therefor. It also provides an intermediate for preparing the compound represented by formula (I), a chiral enantiomer thereof, or a pharmaceutically acceptable salt thereof, and a preparation method therefor. Additionally, the present application provides a pharmaceutical composition comprising the compound represented by formula (I), a chiral enantiomer thereof, or a pharmaceutically acceptable salt thereof, and the pharmaceutical use of the compound represented by formula (I), a chiral enantiomer thereof, or a pharmaceutically acceptable salt thereof, or the pharmaceutical composition comprising any of the above substances. The compounds of the present application exhibit good JAK2 kinase inhibitory activity and high selectivity for JAK2 kinase. Furthermore, the compounds of the present application have significant pharmacokinetic advantages, provide more options for the prevention and/or treatment of diseases (particularly autoimmune diseases, myeloproliferative neoplastic diseases, and graft-versus-host disease) associated with abnormal JAK signaling pathways, and have a good application prospect in clinical practice. In a first aspect, the present application provides a compound represented by formula (I), a chiral enantiomer thereof, or a pharmaceutically acceptable salt thereof: 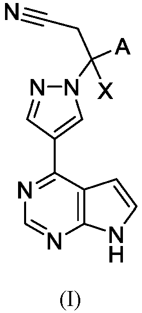 in the formula (I),
A is alkyl or cycloalkyl, wherein the alkyl or the cycloalkyl is optionally substituted with fluoro, alkyl, or cycloalkylene, and the alkyl, the cycloalkyl, or the cycloalkylene has at least one pair of gem-difluoride groups thereon;
X is H or (CH₂)ₙ, wherein n = 0, 1, 2, 3, 4, or 5; when X is (CH₂)ₙ, it is linked to A to form a C₃
-C₇ cycloalkylene, wherein the cycloalkylene is optionally substituted with fluoro or alkyl, and the cycloalkylene or the alkyl has at least one pair of gem-difluoride groups thereon.

In a preferred embodiment, the present application provides a compound represented by formula (II), a chiral enantiomer thereof, or a pharmaceutically acceptable salt thereof: 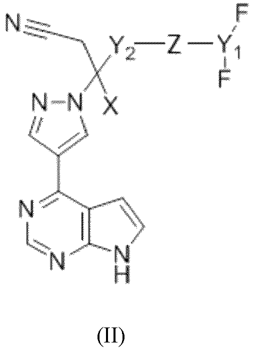 in the formula (II),
Y₁ is CR₁, wherein R₁ is a bond, H, or F;
Z is a bond or (CH₂)ₘ, wherein m = 1, 2, or 3;
Y₂ is alkylene or cycloalkylene, wherein the alkylene or the cycloalkylene is optionally substituted with fluoro, alkyl, or cycloalkylene, or Y₂ is linked to Z or Y₁ to form a C₃-C₇ cycloalkylene; and X is H or (CH₂)ₙ, wherein n = 0, 1, 2, 3, 4, or 5; when X is (CH₂)ₙ, it is linked to Y₁, Z, or Y₂ to form a C₃-C₇ cycloalkylene.

As a preferred embodiment, in the above formula (II):
Y₁ is CR₁, wherein R₁ is a bond;
Z is a bond or (CH₂)ₘ, wherein m = 1, 2, or 3;
Y₂ is alkylene;
X is (CH₂)ₙ, wherein n = 0, 1, 2, 3, 4, or 5; and
X is linked to Y₁ to form a C₃-C₇ cycloalkylene, preferably forming a C₆ cycloalkylene.

As another preferred embodiment, in the above formula (II):
Y₁ is CR₁, wherein R₁ is F;
Z is a bond or (CH₂)ₘ, wherein m = 1, 2, or 3;
Y₂ is alkylene, preferably C₁-C₅ alkylene, more preferably C₂-C₃ alkylene; and
X is H.

As another preferred embodiment, in the above formula (II):
Y₁ is CR₁, wherein R₁ is F;
Z is a bond or (CH₂)ₘ, wherein m = 1, 2, or 3;
Y₂ is alkylene;
X is (CH₂)ₙ, wherein n = 1, 2, 3, 4, or 5; and
X is linked to Z to form a C₃-C₇ cycloalkylene, preferably forming a C₆ cycloalkylene.

As another preferred embodiment, in the above formula (II):
Y₁ is CR₁, wherein R₁ is a bond;
Z is a bond or (CH₂)ₘ, wherein m = 1, 2, or 3;
Y₂ is alkylene and Y₂ is linked to Y₁ to form a C₃-C₇ cycloalkylene, preferably forming a C₄ or C₆ cycloalkylene; and
X is H.

As another preferred embodiment, in the above formula (II):
Y₁ is CR₁, wherein R₁ is F;
Z is (CH₂)ₘ, wherein m = 1, 2, or 3;
Y₂ is alkylene and Y₂ is linked to Z to form a C₃-C₇ cycloalkylene, preferably forming a C₆ cycloalkylene; and
X is H.

As another preferred embodiment, in the above formula (II):
Y₁ is CR₁, wherein R₁ is F;
Z is a bond or (CH₂)ₘ, wherein m = 1, 2, or 3;
Y₂ is alkylene or cycloalkylene, which is optionally substituted with alkyl, cycloalkyl, or cycloalkylene; and
X is H.

In a preferred embodiment, the present application provides a compound represented by any of the following formulas, a chiral enantiomer thereof, or a pharmaceutically acceptable salt thereof:

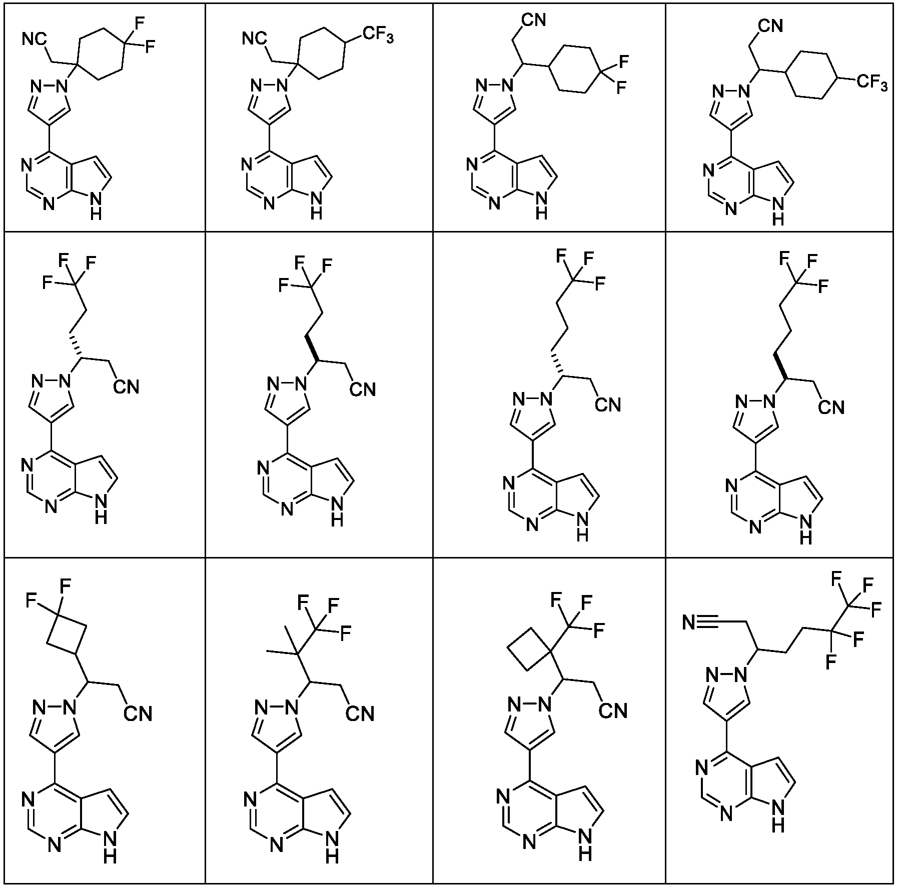

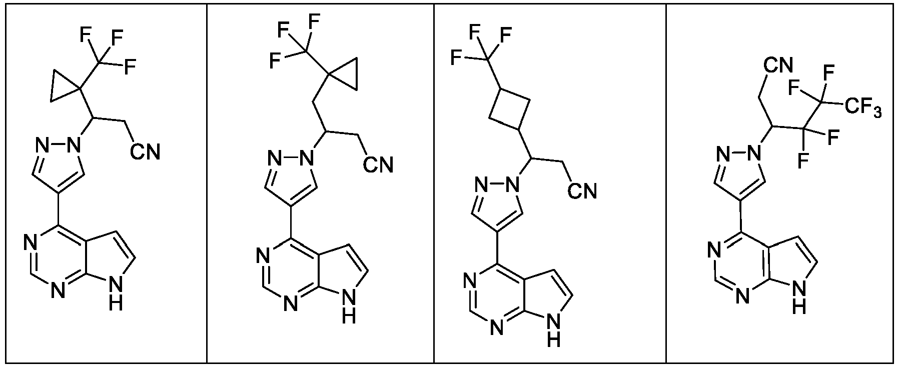

In a second aspect, the present application provides a method for preparing the compound, the chiral enantiomer thereof, or the pharmaceutically acceptable salt thereof according to the first aspect, comprising the following steps:
(1) subjecting compound III-1 and diethyl cyanomethyl phosphate to olefination reaction in the presence of alkali to produce compound III-2;
(2) subjecting the compound III-2 and compound III-3 to Michael addition reaction in the presence of alkali under heating to produce compound III; and
(3) deprotecting the compound III in the presence of alkali to produce compound I;

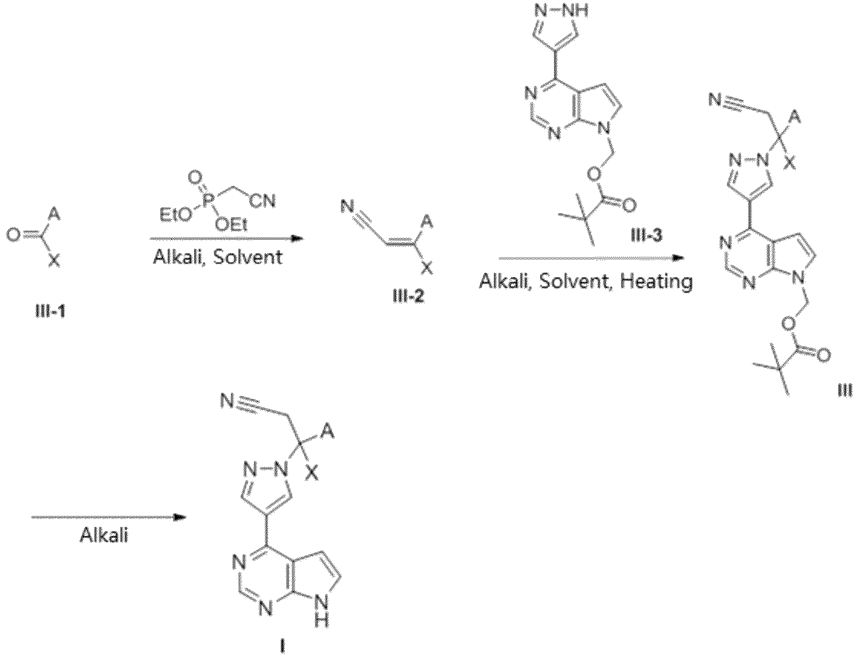

In a preferred embodiment, the alkali used in the step (1) is selected from any one or more of sodium hydride, sodium tert-butoxide, potassium tert-butoxide, lithium tert-butoxide, lithium bromide, lithium chloride, triethylamine, 4-dimethylaminopyridine, cesium carbonate, potassium carbonate, and sodium carbonate; the solvent used in the step (1) is an aprotic solvent, and preferably, the solvent used is selected from any one or more of tetrahydrofuran, dimethylformamide, and dimethyl sulfoxide;
the alkali in the step (2) is selected from any one or more of 1,8-diazabicyclo(5.4.0)undec-7-ene, sodium tert-butoxide, potassium tert-butoxide, triethylamine, 4-dimethylaminopyridine, cesium carbonate, potassium carbonate, sodium carbonate, potassium phosphate, and sodium phosphate; the solvent used in the step (2) is a protic or aprotic solvent, and preferably, the solvent is selected from any one or more of acetonitrile, N-methylpyrrolidone, dimethylformamide, dimethylsulfoxide, tetrahydrofuran, methanol, and ethanol; the heating in the step (2) is performed at a temperature of 52-82°C;
the alkali used in the step (3) is selected from any one or more of sodium hydroxide, lithium hydroxide, and potassium hydroxide.

In a third aspect, the present application provides an intermediate for preparing the compound, the chiral enantiomer thereof, or the pharmaceutically acceptable salt thereof according to the above first aspect, having a structure as shown in general formula (III): 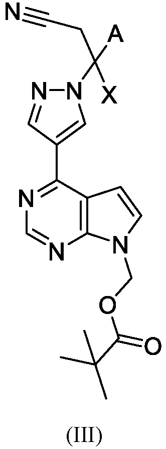 in the formula (III),
A is alkyl or cycloalkyl, wherein the alkyl or the cycloalkyl is optionally substituted with fluoro, alkyl, or cycloalkylene, and the alkyl, the cycloalkyl, or the cycloalkylene has at least one pair of gem-difluoride groups thereon;
X is H or (CH₂)ₙ, wherein n = 0, 1, 2, 3, 4, or 5; when X is (CH₂)ₙ, it is linked to A to form a C₃-C₇ cycloalkylene, wherein the cycloalkylene is optionally substituted with fluoro or alkyl, and the cycloalkylene or the alkyl has at least one pair of gem-difluoride groups thereon.

In a fourth aspect, the present application provides a method for preparing the intermediate according to the above third aspect, comprising the following steps:
(1) subjecting compound III-1 and diethyl cyanomethyl phosphate to olefination reaction in the presence of alkali to produce compound III-2;
(2) subjecting the compound III-2 and compound III-3 to Michael addition reaction in the presence of alkali under heating to produce compound III;

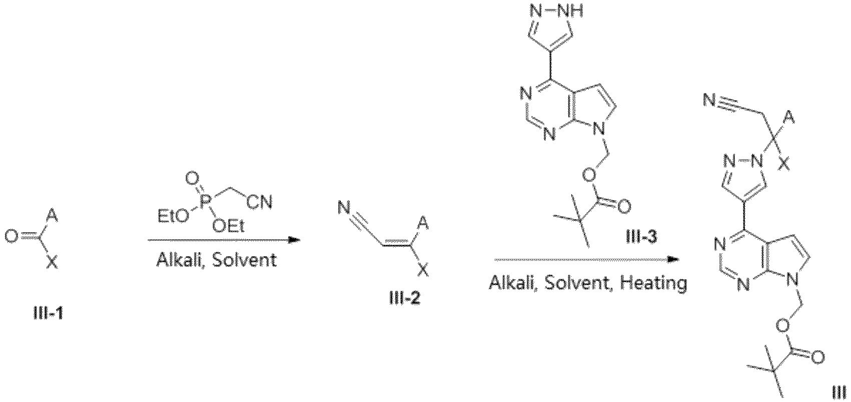

In a preferred embodiment, the alkali used in the step (1) is selected from any one or more of sodium hydride, sodium tert-butoxide, potassium tert-butoxide, lithium tert-butoxide, lithium bromide, lithium chloride, triethylamine, 4-dimethylaminopyridine, cesium carbonate, potassium carbonate, and sodium carbonate; the solvent used in the step (1) is an aprotic solvent, and preferably, the solvent used is selected from any one or more of tetrahydrofuran, dimethylformamide, and dimethyl sulfoxide;
the alkali used in the step (2) is selected from any one or more of 1,8-diazabicyclo(5.4.0)undec-7-ene, sodium tert-butoxide, potassium tert-butoxide, triethylamine, 4-dimethylaminopyridine, cesium carbonate, potassium carbonate, sodium carbonate, potassium phosphate, and sodium phosphate; the solvent used in the step (2) is a protic or aprotic solvent, and preferably, the solvent is selected from any one or more of acetonitrile, N-methylpyrrolidone, dimethylformamide, dimethylsulfoxide, tetrahydrofuran, methanol, and ethanol; and the heating in the step (2) is performed at a temperature of 52-82°C.

In a fifth aspect, the present application provides a pharmaceutical composition comprising a therapeutically effective amount of the compound, the chiral enantiomer thereof, or the pharmaceutically acceptable salt thereof according to the first aspect above, and pharmaceutically acceptable carrier, diluent, and/or excipient.

In a sixth aspect, the present application provides use of the compound, the chiral enantiomer thereof, or the pharmaceutically acceptable salt thereof according to the first aspect, or the pharmaceutical composition according to the fifth aspect in the preparation of a medicament for the prevention and/or treatment of a disease associated with abnormal JAK signaling pathways. Preferably, the disease is an autoimmune disease, a myeloproliferative neoplastic disease, or a graft-versus-host disease.

Further preferably, the autoimmune disease is selected from rheumatoid arthritis, ulcerative colitis, systemic lupus erythematosus, atopic dermatitis, or multiple sclerosis.

Further preferably, the myeloproliferative neoplastic disease is selected from essential thrombocythemia, myelofibrosis, or polycythemia vera.

Further preferably, the graft-versus-host disease is selected from acute graft-versus-host disease or chronic graft-versus-host disease.

### Beneficial effect

1. The compounds of the present application exhibit good JAK2 kinase inhibitory activity and high selectivity for JAK2 kinase;
2. The compounds of the present application have significant pharmacokinetic advantages, provide more options for the prevention and/or treatment of diseases associated with abnormal JAK signaling pathways, and have a good application prospect in clinical practice.

### Detailed Description of the Invention

In order to make the purposes, technical solutions, and advantages of the present application clearer, the technical solutions in the examples of the present application will be clearly and completely described below. Apparently, the described examples are just a part of the examples of the present application, but not all of the examples. Based on the described examples in the present application, all other embodiments obtained by those of ordinary skill in the art without creative work shall fall within the scope of protection of the present application.

In addition, numerous specific details are set forth in the following detailed description in order to better elucidate the present application. Those of ordinary skill in the art will appreciate that the application can be practiced without certain specific details. In some embodiments, materials, methods, etc., which are well known to those skilled in the art, are not described in detail in order to highlight the gist of the present application.

Unless explicitly indicated otherwise, throughout the specification and claims, the term "comprising" or variations thereof such as "including" or "having" or the like intended to denote the inclusion of a stated element, without the exclusion of others.

It should be noted that unless indicated to the contrary, terms used in the specification and claims have the following meanings.

The term "alkyl" refers to a saturated aliphatic hydrocarbon group comprising straight and branched groups containing 1 to 20 carbon atoms, which is preferably alkyl containing 1 to 10 carbon atoms, more preferably alkyl containing 1 to 6 carbon atoms, most preferably alkyl containing 1 to 4 carbon atoms, and most preferably methyl.

Non-limiting examples include methyl, ethyl, n-propyl, isopropyl, n-butyl, isobutyl, *tert*-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof. More preferred is a lower alkyl group containing 1 to 6 carbon atoms, and non-limiting examples include methyl, ethyl, n-propyl, isopropyl, n-butyl, isobutyl, *tert*-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like. The alkyl may be substituted or unsubstituted, and when substituted, a substituent may be at any available linkage point, and preferably the substituent is one or more independently selected from the following groups: alkyl, alkenyl, alkynyl, alkoxy, alkylthio, alkylamino, halogen, mercapto, hydroxyl, nitro, cyano, cycloalkyl, heterocyclic alkyl, aryl, heteroaryl, cycloalkoxy, heterocyclic alkoxy, cycloalkylthio, heterocyclic alkylthio, oxo, amino, haloalkyl, hydroxyalkyl, carboxyl or carboxylic acid ester group.

The term "cycloalkyl" refers to a saturated or partially unsaturated monocyclic or polycyclic hydrocarbon group comprising 3 to 20 carbon atoms, preferably 3 to 12 carbon atoms, more preferably 3 to 10 carbon atoms, most preferably 3 to 6 carbon atoms, and most preferably cyclopropyl or cyclopentyl. Non-limiting examples of a monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like, and preferably cyclopropyl and cyclopentyl. Polycyclic cycloalkyl includes cycloalkyl of a spiro ring, a fused ring, and a bridged ring. The cycloalkyl may be optionally substituted or unsubstituted, and when substituted, the substituent is preferably one or more independently selected from the following groups: alkyl, alkenyl, alkynyl, alkoxy, alkylthio, alkylamino, halogen, mercapto, hydroxyl, nitro, cyano, cycloalkyl, heterocyclic alkyl, aryl, heteroaryl, cycloalkoxy, heterocyclic alkoxy, cycloalkylthio, heterocyclic alkylthio, oxo, amino, haloalkyl, hydroxyalkyl, carboxyl or carboxylic acid ester group.

The term "alkylene" refers to a divalent, straight or branched chain alkane group, which is composed of carbon and hydrogen atoms, is free of unsaturation, and is bonded to one group by a single bond and to the other group (or a ring system) by another single bond, for example, as used herein, "C₁₋₅ alkylene" refers to an alkylene group containing 1 to 5 carbon atoms, "C₂₋₃ alkylene" refers to an alkylene group containing 2 to 3 carbon atoms; and non-limiting examples include methylene (-CH₂-), 1,2-ethylene (-CH₂CH₂-), 1,3-propylene (-CH₂CH₂CH₂-), 1-methyl-1,2-ethylene (-CH(CH₃)CH₂-), 1,4-butylene (-CH₂CH₂CH₂CH₂-), 1-methyl-1,3-propylene (-CH(CH₃)CH₂CH₂-), 1,1-dimethyl-1,2-ethylene (-C(CH₃)₂CH₂-), 1,2-dimethyl-1,2-ethylidene (-CH(CH₃)CH(CH₃)-), etc.

The term "cycloalkylene" refers to a divalent, monocyclic or polycyclic (including bridged and spirocyclic forms), non-aromatic cyclic hydrocarbon group, which composed of only carbon and hydrogen atoms, is free of unsaturation, and is bonded to one group by a single bond and to the other group by another single bond, for example, as used herein, "C₃₋₇ cycloalkylene" refers to a cycloalkylene group containing 3 to 7 carbon atoms, "C₄ cycloalkylene" refers to a cycloalkylene group containing 4 carbon atoms, and "C₆ cycloalkylene" refers to a cycloalkylene group containing 6 carbon atoms; and non-limiting examples include cyclopropane-1,1-ylidene, cyclopropane-1,2-ylidene, cyclobutane-1,1-ylidene, cyclobutane-1,2-ylidene, cyclobutane-1,3-ylidene, etc.

The term "bond" refers to the chemical bonding of two atoms or moieties (i.e., groups, fragments) in which the atoms bonded by the bond are considered to be part of a larger substructure.

The term "chiral enantiomer" refers to two chiral molecules that are mirror images but cannot be superimposed on each other.

The term "protecting group" refers to a group used to block the reactivity of a functional group. Examples of protecting groups include, but are not limited to, methylene pivalate.

The terms "optional" or "optionally" mean that the subsequently described event or circumstance may, but does not have to, occur, and that the description includes instances where the event or circumstance does and instances where it does not occur. For example, a "heterocycloalkyl group optionally substituted with an alkyl group" means that an alkyl group may be present, but is not mandatory, and the description includes both cases where the heterocycloalkyl group is substituted with an alkyl group and cases where the heterocycloalkyl group is not substituted with an alkyl group.

The term "substituted" means that one or more hydrogen atoms, preferably up to 5, more preferably 1 to 3 hydrogen atoms, within the group are substituted, independently of one another, by a corresponding number of substituents. It goes without saying that the substituents only occupy their feasible chemical positions and that a person skilled in the art can readily determine (experimentally or theoretically) the feasible or infeasible substitutions without undue experimentation. For example, an amino or hydroxyl group having a free hydrogen may be unstable when combined with a carbon atom bearing an unsaturated bond (e.g., olefinic).

Unless otherwise specified, the instruments, consumables, and reagents used in the following examples can be obtained by conventional commercial means, and the experimental methods not specified in the examples should be chosen according to the conventional methods and conditions, or according to the manufacturer's instructions.

### Preparation of the compound represented by formula (I) and intermediate thereof according to the present application.

The intermediate of the compound represented by formula (I) can be synthesized according to the following general synthetic route: 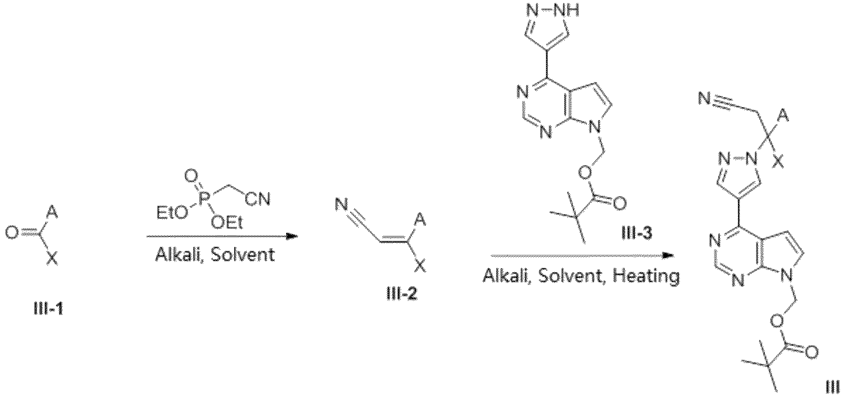 which specifically comprises the following steps:
(1) subjecting compound III-1 and diethyl cyanomethyl phosphate to olefination reaction in the presence of alkali to produce compound III-2;
   preferably, the alkali is selected from any one or more of sodium hydride, sodium tert-butoxide, potassium tert-butoxide, lithium tert-butoxide, lithium bromide, lithium chloride, triethylamine, 4-dimethylaminopyridine, cesium carbonate, potassium carbonate, and sodium carbonate; preferably, the solvent used is selected from any one or more of tetrahydrofuran, dimethylformamide, and dimethyl sulfoxide;
(2) subjecting the compound III-2 and compound III-3 to Michael addition reaction in the presence of alkali under heating to produce compound III;
preferably, the alkali is selected from any one or more of 1,8-diazabicyclo(5.4.0)undec-7-ene, sodium tert-butoxide, potassium tert-butoxide, triethylamine, 4-dimethylaminopyridine, cesium carbonate, potassium carbonate, sodium carbonate, potassium phosphate, and sodium phosphate; preferably, the solvent used is selected from any one or more of acetonitrile, N-methylpyrrolidone, dimethylformamide, dimethylsulfoxide, tetrahydrofuran, methanol, and ethanol; and the heating is performed at a temperature of 52-82°C.

The compound represented by formula (I) in the present application can be synthesized according to the following general synthetic route: 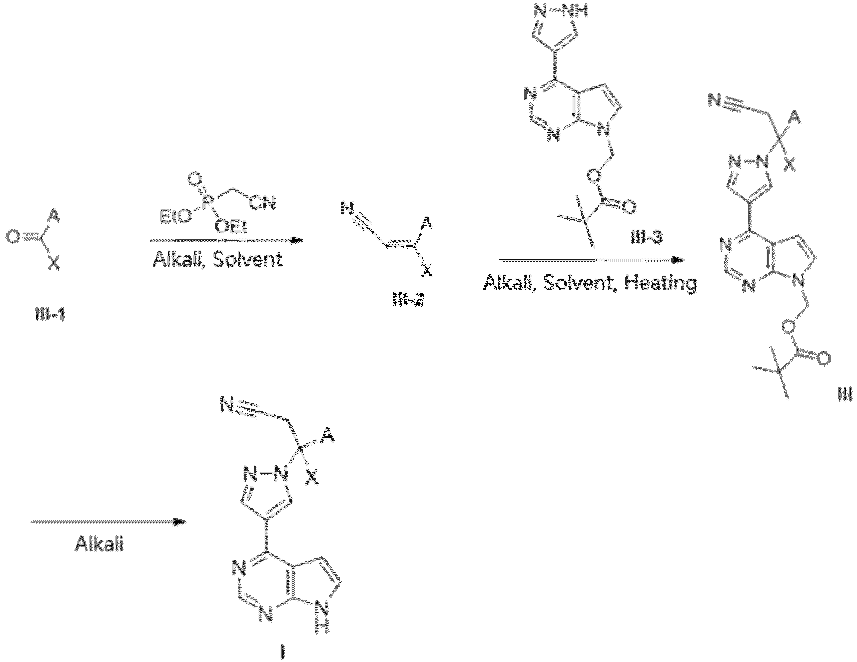 which specifically comprises the following steps:
(1) preparing compound III-2 as described above;
(2) preparing compound III as described above; and
(3) deprotecting the compound III in the presence of alkali to produce compound I; preferably, the alkali is selected from any one or more of sodium hydroxide, lithium hydroxide, and potassium hydroxide.

Some specific preparation examples of the compounds of formula (I) of the present application and their JAK2/JAK3 kinase inhibitory activity tests and pharmacokinetic tests are provided below.

### Example 1. Synthesis of Compound 1

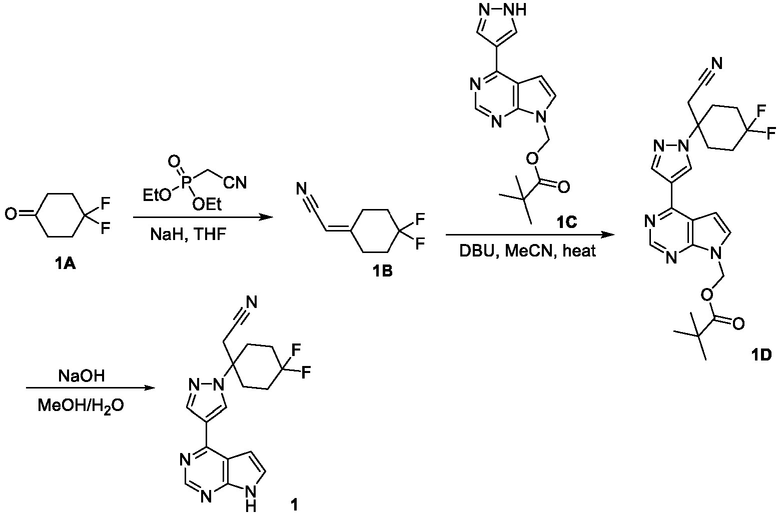

**The procedure comprises specific steps as follows:**
Step (1):
   Diethyl cyanomethyl phosphate (1.95 g, 11 mmol) was dissolved in dry tetrahydrofuran (50 mL), sodium hydride (420 mg, 13 mmol) was added under ice bath, and after stirring for one hour, Compound 1A (1.34 g, 10 mmol) was added. The mixture was left to react overnight with stirring at room temperature. The reaction system was quenched by the addition of saturated ammonium chloride solution (20 mL), and extracted with ethyl acetate (20 mL × 3). The organic phases were combined, washed with saturated brine (20 mL), dried over anhydrous sodium sulfate, and filtered. The filtrate was concentrated under reduced pressure to give a residue, namely Compound 1B (LCMS (ESI +): 158.08 (M + H)⁺), which was used directly in the next step.
Step (2):
   Compound 1C (1.35 g, 5 mmol), Compound 1B (15 mmol) obtained in step (1), and 1,8-diazabicyclo(5.4.0)undec-7-ene (5 mmol) were dissolved in acetonitrile (30 mL), and the mixture was heated to 80°Cto react for 5 days. Aft cooling to room temperature, the reaction mixture was concentrated to remove most of acetonitrile, and extracted with water (20 mL) and ethyl acetate (10 mL×3). The organic phases were combined, washed with saturated saline (20 mL), dried over anhydrous sodium sulfate, and filtered. The filtrate was concentrated under reduced pressure, and the residue was purified by column chromatography (dichloromethane: methanol = 100: 0-100: 5) to give Compound 1D (LCMS (ESI +): 457.22 (M + H)⁺).
Step (3):
   Compound 1D (912 mg, 2 mmol) obtained in step (2) was dissolved in methanol and water (5/5 mL), and sodium hydroxide (160 mg, 4 mmol) was added. The mixture was left to react at room temperature, and the reaction was monitored by TLC until the starting materials were consumed. The reaction mixture was extracted with ethyl acetate (10 mL × 3). The organic phases were combined, washed with saturated saline (20 mL), dried over anhydrous sodium sulfate, and filtered. The filtrate was concentrated under reduced pressure, and the residue was purified by column chromatography (dichloromethane: methanol = 100: 0-100: 5) to give Compound 1. (LCMS (ESI +): 343.15 (M + H)⁺).

### Example 2. Synthesis and Resolution of Compound 2

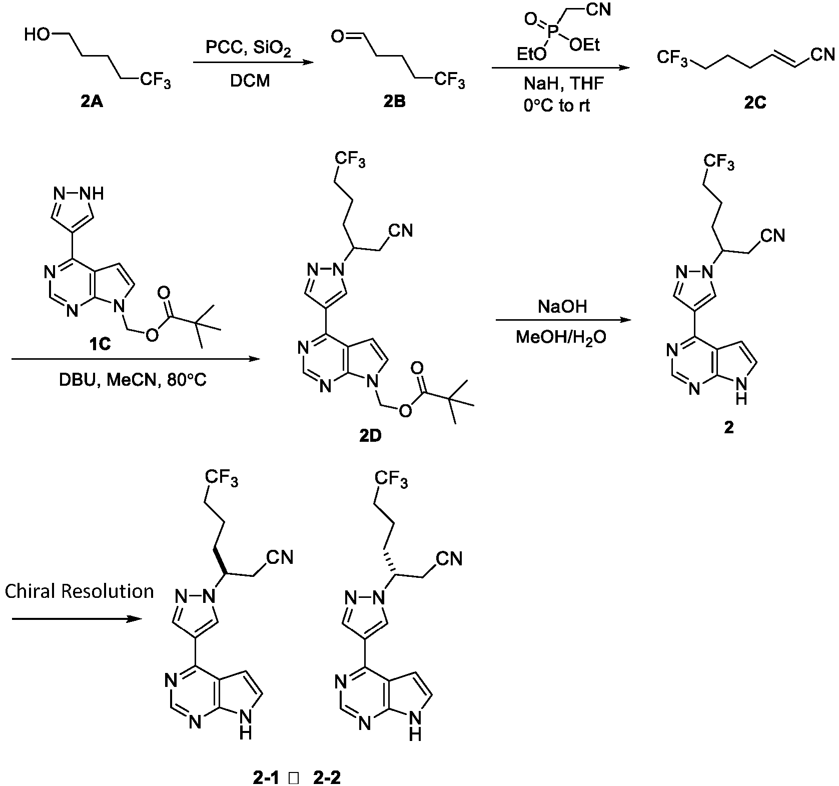

**The procedure comprises specific steps as follows:**
Step (1):
   Compound **2A** (1.42 g, 10 mmol) was dissolved in dichloromethane (20 mL), and silica gel powder (SiO₂, 3 g) and pyridine chlorochromate (3.24 g, 15 mmol) were added. The mixture was stirred at room temperature overnight, followed by filtering with diatomite. The filtrate was concentrated to give Compound **2B** (LCMS (ESI +): 141.05 (M + H)⁺), which was used directly in the next step.
Step (2):
   Diethyl cyanomethyl phosphate (1.95 g, 11 mmol) was dissolved in dry tetrahydrofuran (50 mL), sodium hydride (420 mg, 13 mmol) was added under ice bath, and after stirring for one hour, Compound **2B** (1.4 g, 10 mmol) was added. The mixture was left to react overnight with stirring at room temperature. The reaction system was quenched by the addition of saturated ammonium chloride solution (20 mL), and extracted with ethyl acetate (20 mL × 3). The organic phases were combined, washed with saturated brine (20 mL), dried over anhydrous sodium sulfate, and filtered. The filtrate was concentrated under reduced pressure to give a residue, namely Compound 2C (LCMS (ESI +): 164.07 (M + H)⁺), which was used directly in the next step.
Step (3):
   Compound **1C** (1.35 g, 5 mmol), Compound **2C** (15 mmol) obtained in step (2), and 1,8-diazabicyclo(5.4.0)undec-7-ene (5 mmol) were dissolved in acetonitrile (30 mL), and the mixture was heated to 80°Cto react for 5 days. Aft cooling to room temperature, the reaction mixture was concentrated to remove most of the acetonitrile, extracted with water (20 mL) and ethyl acetate (10 mL × 3). The organic phases were combined, washed with saturated saline (20 mL), dried over anhydrous sodium sulfate, and filtered. The filtrate was concentrated under reduced pressure, and the residue was purified by column chromatography (dichloromethane: methanol = 100: 0-100: 5) to give Compound 2D (LCMS (ESI +): 463.21 (M + H)⁺).
Step (4):
   Compound **2D** (924 mg, 2 mmol) obtained in step (3) was dissolved in methanol and water (5/5 mL), and sodium hydroxide (160 mg, 4 mmol) was added. The mixture was left to react at room temperature, and the reaction was monitored by TLC until the starting materials were consumed. The reaction mixture was extracted with ethyl acetate (10 mL × 3). The organic phases were combined, washed with saturated saline (20 mL), dried over anhydrous sodium sulfate, and filtered. The filtrate was concentrated under reduced pressure, and the residue was purified by column chromatography (dichloromethane: methanol = 100: 0-100: 5) to give Compound 2. (LCMS (ESI +): 349.14 (M + H)⁺).

### Resolution of Compound 2

Resolution conditions: Compound 2 was dissolved in ethanol (at a concentration of 6 mg/mL), and then was subjected to a resolution on a Chiral column UniChiral CND-H(50mm I.D x 250 mmL) with n-hexane/ethanol (80/20, V/V) as mobile phase at a flow rate of 120 mL/min; the UV detection wavelength was 254 nm; and the column temperature was 30°C. After the resolution, compound 2-1 and compound 2-2 were obtained, respectively.

Retention time: Compound 2-1: 8.493 min (> 98% ee), Compound 2-2: 9.994 min (> 98% ee).

### Example 3. Synthesis of Compound 3

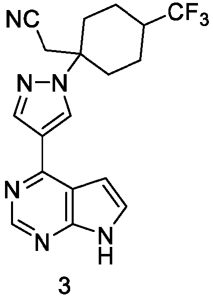

Compound 3 was prepared according to the synthetic procedure of Example 1 with 4-trifluoromethylcyclohexane-1-one as the starting material. LCMS (ESI +): 375.15 (M + H)⁺.

### Example 4. Synthesis of Compound 4

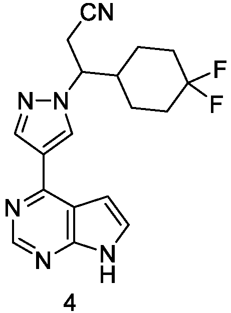

Compound 4 was prepared according to the synthetic procedure of Example 1 with 4,4-difluorocyclohexylformaldehyde as the starting material. LCMS (ESI +): 357.16 (M + H)⁺.

### Example 5. Synthesis of Compound 5

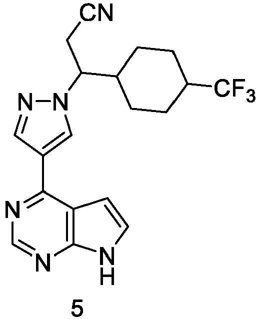

Compound 5 was prepared according to the synthetic procedure of Example 2 with 4-(trifluoromethyl) cyclohexylmethanol as the starting material. LCMS (ESI +): 389.17 (M + H)⁺.

### Example 6. Synthesis and Resolution of Compound 6

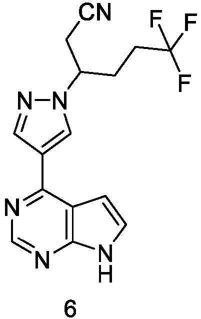

Compound 6 was prepared according to the synthetic procedure of Example 1 with 4,4,4-trifluorobutyraldehyde as the starting material. LCMS (ESI +): 335.12 (M + H)⁺.

### Resolution of Compound 6

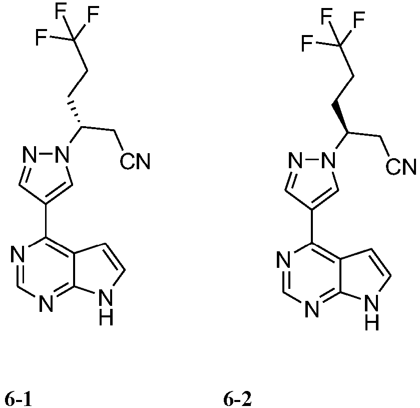

Resolution conditions: Compound 6 was dissolved in n-hexane/ethanol = 80/20 (V/V) (at a concentration of 10 mg/mL), and then was subjected to a resolution on a Chiral column ChiralCel OD-H (0.46cm I.D x 25 cmL) with n-hexane/ethanol (80/20, V/V) as mobile phase at a flow rate of 1.0 mL/min; the UV detection wavelength was 254 nm; and the column temperature was 30°C. Retention time: Compound 6-1: 9.295 min (> 98% ee), Compound 6-2: 11.252 min (> 98% ee).

### Example 7. Synthesis of Compound 7

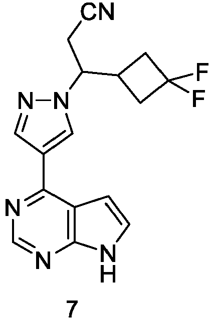

Compound 7 was prepared according to the synthetic procedure of Example 2 with (3,3-difluorocyclobutyl) methanol as the starting material. LCMS (ESI +): 329.13 (M + H)⁺.

### Example 8. Synthesis of Compound 8

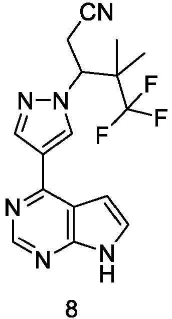

Compound 8 was prepared according to the synthetic procedure of Example 2 with 3,3,3-trifluoro-2,2-dimethylpropan-1-ol as the starting material. LCMS (ESI +): 349.14 (M + H)⁺.

### Example 9. Synthesis of Compound 9

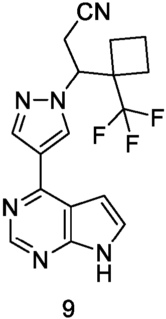

Compound 9 was prepared according to the synthetic procedure of Example 2 with 1-(trifluoromethyl) -1-cyclobutyl-1-methanol as the starting material. LCMS (ESI +): 361.14 (M + H)⁺.

### Example 10. Synthesis of Compound 10

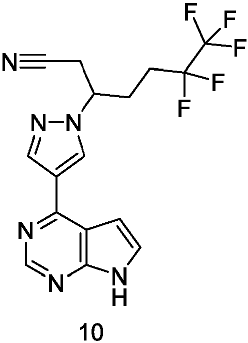

Compound 10 was prepared according to the synthetic procedure of Example 2 with 4,4,5,5,5-pentafluoropentanol as the starting material. LCMS (ESI +): 385.12 (M + H)⁺.

### Example 11. Preparation of Other Compounds

Examples of preparation of other specific compounds include:

**Table 1 Preparation and Characterization of Other Compounds of the Present Application**

| **Compound No.** | **Structural formula** | **Structural characterization** | **Synthesis method** |
|---|---|---|---|
| 11 | 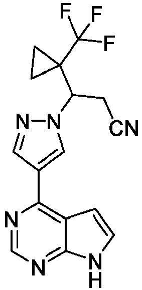 | LCMS (ESI +): 347.15 (M + H)⁺ | Compound 11 was prepared according to the synthetic procedure of Example 2 with 1-(trifluoromethyl) cyclopropanemethanol as the starting material. |
| 12 | 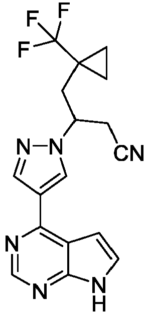 | LCMS (ESI +): 361.17 (M + H)⁺ | Compound 12 was prepared according to the synthetic procedure of Example 2 with 2-(1-(trifluoromethyl) cyclopropyl) ethane-1-ol as the starting material. |
| 13 | 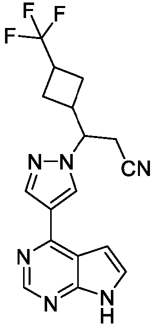 | LCMS (ESI +): 361.17 (M + H)⁺ | Compound 13 was prepared according to the synthetic procedure of Example 2 with (3-(trifluoromethyl) cyclobutyl) methanol as the starting material. |
| 14 | 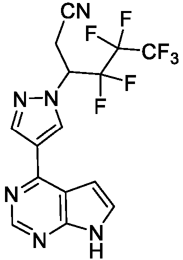 | LCMS (ESI +): 407.07 (M + H)⁺ | Compound 14 was prepared according to the synthetic procedure of Example 2 with 2,2,3,3,4,4,4-heptafluorobuta n-1-ol as the starting material. |

### Example 12. Detection of JAK2/JAK3 Kinase Inhibitory Activity

1. Experimental consumables

| | | | |
|---|---|---|---|
| JAK2: | Carna | 09-045 | 14CBS-0374 H |
| JAK3: | Carna | 08-046 | 19CBS-0798 B |
| ATP (10 mM): | CST | 9804 | |
| DTT: | 100 mM | | |
| MgCl₂: | 1 M | | |

TK substrate-biotin ( hereinafter referred to as substrate): Cisbio, # 61TK0BLC*
Streptavidin-XL665: Cisbio, # 610SAXLG*
HTRF Kinase-TK kit : Cisbio, # 62TK0PEC
TK-Antibody-Eu³-Cryptate : from the kit Cisbio, # 62TK0PEC
HTRF 96 well low volume plate : Cisbio, # 66PL96001
Ruxolitinib: from Chongqing Gongzhou Pharmatech Co., Ltd., CAS No.: 941678-49-5

2. Detection conditions
JAK2: 0.008 ng/µL, ATP 4 µM, substrate 1 µM, time 2 h
JAK3: 0.1 ng/µL, ATP 3 µM, substrate 1 µM, time 3 h

3. JAK2 kinase inhibitory activity test

### 3.1 Reagent preparation

1) Preparation of 1 × kinase buffer: 5 × kinase buffer was diluted with sterile water to 1 × kinase buffer, then 5 mM MgCl₂ , and 1 mM DTT were added.
2) Preparation of 5 × JAK2: JAK2 solution with a concentration of 166 ng/µL was diluted to a final concentration of 5 ×, i.e., 0.04 ng/µL. Specifically, a solution of JAK2 was diluted to 1.66 ng/µL first, and then diluted 41.5 times from 1.66 ng/µL to 0.04 ng/µL.
3) Preparation of 5 × ATP: 4 µM ATP was formulated from its 5 × solution, i.e., 20 µM, and the required ATP concentration was obtained by 500-fold dilution of 10 mM ATP.
4) Preparation of 5 × substrate: 5 × 1 µM is namely 5 µM; the substrate solution with a concentration of 500 µM was subjected to 100-fold dilution to give 5 µM of a substrate solution.
5) Preparation of 2.5 × compound to be tested: the buffer solution concentration of compound to be tested was 10 mM. The treatment concentration was starting from 10 µM. A buffer solution of 100 ×, namely 1 mM, was firstly prepared by 10-fold dilution of 10 mM, and then diluted by a gradient of 1:3, with totally 10 concentrations being prepared. 2 µL of the diluted solution of compound to be tested was added to 78 µL of 1 × kinase buffer to give 2.5 × the compound to be tested.
   In addition, 2 µL of DMSO was pipetted to 78 µL of 1 × kinase buffer to give 2.5 × DMSO.
6) Preparation of 1 µM Streptavidin-XL665: Streptavidin-XL665 with a concentration of 16.67 µM was diluted 16.67-fold with detection buffer prior to use.
7) Preparation of 1 × TK-Antibody-Eu³-Cryptate: TK-Antibody-Eu³-Cryptate stock solution, which was 100 × solution, was diluted to 1 × solution with detection buffer prior to use.

### 3.2 Test method

1) Treatment wells of the compound to be tested (T-compound): In HTRF 96 well low volume plate, 4 µL of 2.5 × the compound to be tested described above was added to a well, followed by the addition of 2 µL of 5 × substrate to one side of the well and the addition of 2 µL of 5 × JAK2 to the other side of the well.
   DMSO control wells without the compound to be tested (T-enzyme): In HTRF 96 well low volume plate, 4 µL of 2.5 × DMSO described above was added to a well, followed by the addition of 2 µL of 5 × substrate to one side of the well and the addition of 2 µL of 5 × JAK2 to the other side of the well.
   Enzyme-free blank control (T-without enzyme): In HTRF 96 well low volume plate, 4 µL of 2.5 × DMSO described above was added to a well, followed by the addition of 2 µL of 5 × substrate to one side of the well and the addition of 2 µL of 1 × kinase buffer to the other side of the well.
2) The plate was sealed with a plate sealing film, placed into a centrifuge, and centrifuged at 1000 rpm for 2 minutes.
3) 2 µL of 5 × ATP was added into each well, and the plate was sealed with a plate sealing film, centrifuged at 1000 rpm for 1 minute, and then incubated in an incubator at 30°C for 2 h.
4) After the incubation, Streptavidin-XL665 and 1 × TK-Antibody-Eu³-Cryptate described above were mixed at a ratio of 1:1, 10 µL of the mixture was added into each well, and then the plate was centrifuged at 1000 rpm for 1 minute.
5) The plate was put back into the incubator and incubated for another 1 h. After the incubation, HTRF 620/665 signals were read on a multifunctional microplate reader.

### 4. JAK3 kinase inhibitory activity test

### 4.1 Reagent preparation

1) Preparation of 1 × kinase buffer: 5 × kinase buffer was diluted with sterile water to 1 × kinase buffer, and then 5 mM MgCl₂, and 1 mM DTT were added.
2) Preparation of 5 × JAK3: JAK3 solution with a concentration of 124 ng/µL was diluted 248-fold to a final concentration of 5 ×, i.e. 0.5 ng/µL.
3) Preparation of 5 × ATP: 3 µM ATP was formulated from its 5 × solution, i.e., 15 µM, and the required ATP concentration was obtained by 666.67-fold dilution of 10 mM ATP.
4) Preparation of 5 × substrate: 5 ×1 µM is namely 5 µM; the substrate solution with a concentration of 500 µM was subjected to 100-fold dilution to give 5 µM of a substrate solution.
5) Preparation of 2.5 × compound to be tested: the buffer solution concentration of compound to be tested was 10 mM. The treatment concentration was starting from 10 µM. A buffer solution of 100 ×, namely 1 mM, was firstly prepared by 10-fold dilution of 10 mM, and then diluted by a gradient of 1:3, with totally 10 concentrations being prepared. 2 µL of the diluted solution of compound to be tested was added to 78 µL of 1 × kinase buffer to give 2.5 × the compound to be tested.
   In addition, 2 µL of DMSO was pipetted to 78 µL of 1 × kinase buffer to give 2.5 × DMSO.
6) Preparation of 1 µM Streptavidin-XL665: Streptavidin-XL665 with a concentration of 16.67 µM was diluted 16.67-fold with detection buffer prior to use.
7) Preparation of 1 × TK-Antibody-Eu³-Cryptate: TK-Antibody-Eu³-Cryptate stock solution, which was 100 × solution, was diluted to 1 × solution with detection buffer prior to use.

### 4.2 Test method

1) Treatment wells of the compound to be tested (T-compound): In HTRF 96 well low volume plate, 4 µL of 2.5 × the compound to be tested described above was added to a well, followed by the addition of 2 µL of 5 × substrate to one side of the well and the addition of 2 µL of 5 × JAK3 to the other side of the well.
   DMSO control wells without the compound to be tested (T-enzyme): In HTRF 96 well low volume plate, 4 µL of 2.5 × DMSO described above was added to a well, followed by the addition of 2 µL of 5 × substrate to one side of the well and the addition of 2 µL of 5 × JAK3 to the other side of the well.
   Enzyme-free blank control (T-without enzyme): In HTRF 96 well low volume plate, 4 µL of 2.5 × DMSO described above was added to a well, followed by the addition of 2 µL of 5 × substrate to one side of the well and the addition of 2 µL of 1 × kinase buffer to the other side of the well.
2) The plate was sealed with a plate sealing film, placed into a centrifuge, and centrifuged at 1000 rpm for 2 minutes.
3) 2 µL of 5 × ATP was added into each well, and the plate was sealed with a plate sealing film, centrifuged at 1000 rpm for 1 minute, and then incubated in an incubator at 30°C for 3 h.
4) After the incubation, Streptavidin-XL665 and 1 × TK-Antibody-Eu³-Cryptate described above were mixed at a ratio of 1:1, 10 µL of the mixture was added into each well, and then the plate was centrifuged at 1000 rpm for 1 minute.
5) The plate was put back into the incubator and incubated for another 1 h. After the incubation, HTRF 620/665 signals were read on a multifunctional microplate reader.

### 5. Calculation of inhibition rate and fitting of IC50

Inhibition rate = (T-enzyme - T-compound)/ (T-enzyme - T-without enzyme) × 100% Half-maximal inhibitory concentration (IC50) was fitted using GraphPad Prism 6 based on the inhibition rate of the compound to be tested on the kinase at different concentrations.

Some representative compounds of the present application and the positive control drug, Ruxolitinib, were tested for their inhibitory activity against JAK2 and JAK3 kinases by the above tests. The measured IC50 values are shown in Table 2 below.

**Table 2 IC50s for inhibition of JAK2 and JAK3 kinases by the representative compounds of the present application and Ruxolitinib**

| Compound No. | JAK2 IC50 (nM) | JAK3 IC50 (nM) | JAK2 selectivity (JAK3/JAK2) |
|---|---|---|---|
| 1 | 2.8 | 241.2 | 86 |
| 2-1 | 3.3 | 121.5 | 37 |
| 2-2 | 2.7 | 61.4 | 23 |
| 3 | 1.9 | 151.4 | 80 |
| 4 | 11.1 | 402.3 | 36 |
| 5 | 5.6 | 488.2 | 87 |
| 6-1 | 8.0 | 310.6 | 39 |
| 6-2 | 1.1 | 115.5 | 105 |
| 7 | 4.3 | 478.9 | 111 |
| 8 | 15.0 | 419.4 | 28 |
| 9 | 13.3 | 400.8 | 30 |
| Ruxolitinib | 1.3 | 37.8 | 29 |

It can be seen from Table 2 that the compounds of the present application have a good JAK2 kinase inhibitory activity, and a significant selectivity for JAK2 which is equal to or better than that of the positive control drug Ruxolitinib.

### Example 13. Pharmacokinetic Test

### 1. Experimental animals

Three healthy male C57 mice, aged 6-8 weeks, were purchased from Shanghai Sippr/BK Laboratory Animal Co., Ltd. (Shanghai, China).

### 2. Experimental methods

Mice were fasted overnight prior to oral administration, and food supply was resumed 4 hours after administration with free access to water. Mice were administered at 10 mg compound/kg body weight by gavage, and whole blood samples were collected from the facial vein by semi-serial blood sampling. At 0.125 h, 0.25 h, 0.5 h, 1 h, 2 h, 4 h, 8 h, and 24 h after the administration, respectively, about 30 µL of blood was collected from the tested animal, and was placed in a test tube containing the anticoagulant heparin sodium which was placed on ice until centrifugation; the centrifugation was performed within 15 min at 6800 g for 6 min at 6-8°C. Plasma was transferred to an appropriately labeled tube within 1 hour of blood collection/centrifugation, and stored frozen at approximately -80°C.

### 3. Chromatographic and Mass Spectrometric Conditions

Chromatographic column: Luna^{®}Omega ACQUITY UPLC BEH C18 (2.1 × 50mm, 1.7µm); mobile phase A: H₂O-0.1% FA, mobile phase B: ACN-0.1% FA, flow rate: 0.80 mL/min; gradient elution procedure: initial, 10% B; 0.6 min, 10% B; 1.0 min, 90% B; 1.11 min, 90% B; 1.40 min, 10% B; column temperature: 40°C, injection volume: 2 µL.

Mass spectrometry method: LC-MS/MS-19 (TQ5500) (SCIEX, USA), ion source: ESI source, detection mode: positive ion detection, scanning mode: multiple reaction monitoring (MRM), m/z: 271.10/172.00 Da (tolbutamide, internal standard).

### 4. Preparation of plasma samples

10 µL of plasma sample was added with 200 µL of internal standard working solution (tolbutamide, 100 ng/mL), vortexed for 1 min, and centrifuged for 10 min at 18000 g. 200 µL of a supernatant was transferred to HTRF 96 well low volume plate, and 1 µL of the supernatant was used for LC-MS/MS analysis.

### 5. Result analysis

Pharmacokinetic (PK) parameters were calculated using Phoenix WinNonlin 7.0 software. The oral pharmacokinetic parameters of mice, including AUC, Cₘₐₓ, Tₘₐₓ, T1/2, etc., were estimated by non-compartmental models. The results of oral pharmacokinetic parameters of the representative compounds of the present application (prepared from the above examples, in which compound 2 was a mixture of compound 2-1 and compound 2-2) and the positive control drug Ruxolitinib in mice are shown in Table 3.

**Table 3 Oral pharmacokinetic parameters of the representative compounds of the present application and Ruxolitinib**

| Comp ound No. | Dose mg/kg | T1/2 (h) | Tₘₐₓ (h) | Cₘₐₓ (ng/mL ) | AUC₍₀₋ₜ₎ (h*ng/m L) | AUC_{(0-∞)} (h*ng/m L) | AUC_{(0-∞)} d.n. (h*ng/mL) * | MRT (0-t) (h) | MRT (0-∞) (h) |
|---|---|---|---|---|---|---|---|---|---|
| 1 | 10.0 | 1.18 | 0.33 | 1479.8 0 | 2131.80 | 2152.50 | 215.25 | 1.25 | 1.35 |
| 2 | 10.0 | 0.75 | 0.13 | 1940.3 9 | 1677.31 | 1678.79 | 167.88 | 0.84 | 0.84 |
| 5 | 10.0 | 1.36 | 0.25 | 1407.0 0 | 1978.00 | 1999.00 | 199.90 | 1.22 | 1.31 |
| 6-1 | 10.0 | 0.84 | 0.25 | 6323.0 0 | 9226.00 | 9237.00 | 923.70 | 1.14 | 1.15 |
| 6-2 | 10.0 | 0.74 | 0.08 | 6773.0 0 | 4765.00 | 4769.00 | 476.90 | 0.60 | 0.61 |
| 7 | 10.0 | 1.49 | 0.08 | 3190.0 0 | 1659.00 | 1662.00 | 166.20 | 0.49 | 0.51 |
| 10 | 10.0 | 0.76 | 0.25 | 4410.0 0 | 4482.00 | 4486.00 | 448.60 | 0.91 | 0.92 |
| Ruxolitinib | 5.0 | 0.50 | 0.13 | 893.20 | 577.76 | 579.65 | 115.93 | 0.70 | 0.71 |

| | | | | | | | | | |
|---|---|---|---|---|---|---|---|---|---|
| *d.n. means dose normalization. | | | | | | | | | |

It can be seen from Table 3 that the half-life (T1/2) and exposure amount per unit dose (AUC _{(0-∞)} d.n.) of the compounds of the present application are significantly higher than those of the positive control drug Ruxolitinib, and thus the compounds of the present application have obvious pharmacokinetic advantages.

Finally, it should be noted that the above embodiments are only used to illustrate the technical solutions of the present application, but not to limit it. Although the present application has been described in detail with reference to the foregoing embodiments, those of ordinary skill in the art should understand that the technical solutions described in the foregoing embodiments can still be modified, or some of the technical features thereof can be equivalently replaced. These modifications or replacements do not make the essence of the corresponding technical solutions deviate from the spirit and scope of the technical solutions of the embodiments of the present application.

### Industrial applicability

The compounds containing gem-difluoride group as represented by formula (I) according to the present application exhibit good JAK2 kinase inhibitory activity and high selectivity for JAK2 kinase. In addition, the compounds of the present application have significant pharmacokinetic advantages, provide more options for the prevention and/or treatment of diseases associated with abnormal JAK signaling pathways, and have a good application prospect in clinical practice.

## Claims

1. A compound represented by formula (I), a chiral enantiomer thereof, or a pharmaceutically acceptable salt thereof: 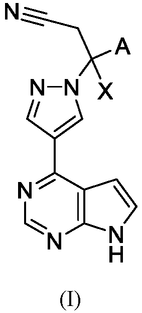 in the formula (I),
A is alkyl or cycloalkyl, wherein the alkyl or the cycloalkyl is optionally substituted with fluoro, alkyl, or cycloalkylene, and the alkyl, the cycloalkyl, or the cycloalkylene has at least one pair of gem-difluoride groups thereon;
X is H or (CH₂)ₙ, wherein n = 0, 1, 2, 3, 4 or 5; when X is (CH₂)ₙ, it is linked to A to form a C₃-C₇ cycloalkylene, wherein the cycloalkylene is optionally substituted by fluoro or alkyl, and the cycloalkylene or the alkyl has at least one pair of gem-difluoride groups thereon.

2. The compound, the chiral enantiomer thereof, or the pharmaceutically acceptable salt thereof according to claim 1, having a structure represented by formula (II): 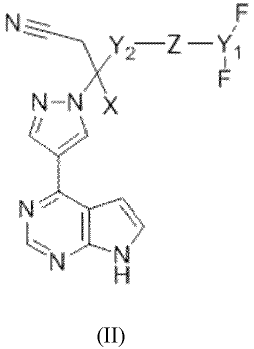 in the formula (II),
Y₁ is CR₁, wherein R₁ is a bond, H, or F;
Z is a bond or (CH₂)ₘ, wherein m = 1, 2, or 3; and
Y₂ is alkylene or cycloalkylene, wherein the alkylene or the cycloalkylene is optionally substituted with fluoro, alkyl, or cycloalkylene, or Y₂ is linked to Z or Y₁ to form a C₃-C₇ cycloalkylene; and
X is H or (CH₂)ₙ, wherein n = 0, 1, 2, 3, 4, or 5; when X is (CH₂)ₙ, it is linked to Y₁, Z, or Y₂ to form a C₃-C₇ cycloalkylene.

3. The compound, the chiral enantiomer thereof, or the pharmaceutically acceptable salt thereof according to claim 2, wherein
Y₁ is CR₁, wherein R₁ is a bond;
Z is a bond or (CH₂)ₘ, wherein m = 1, 2, or 3;
Y₂ is alkylene;
X is (CH₂)ₙ, wherein n = 0, 1, 2, 3, 4, or 5; and
X is linked to Y₁ to form a C₃-C₇ cycloalkylene, preferably forming a C₆ cycloalkylene.

4. The compound, the chiral enantiomer thereof, or the pharmaceutically acceptable salt thereof according to claim 2, wherein
Y₁ is CR₁, wherein R₁ is F;
Z is a bond or (CH₂)ₘ, wherein m = 1, 2, or 3;
Y₂ is alkylene, preferably C₁-C₅ alkylene, more preferably C₂-C₃ alkylene; and
X is H.

5. The compound, the chiral enantiomer thereof, or the pharmaceutically acceptable salt thereof according to claim 2, wherein
Y₁ is CR₁, wherein R₁ is F;
Z is a bond or (CH₂)ₘ, wherein m = 1, 2, or 3;
Y₂ is alkylene;
X is (CH₂)ₙ, wherein n = 1, 2, 3, 4, or 5; and
X is linked to Z to form a C₃-C₇ cycloalkylene, preferably forming a C₆ cycloalkylene.

6. The compound, the chiral enantiomer thereof, or the pharmaceutically acceptable salt thereof according to claim 2, wherein
Y₁ is CR₁, wherein R₁ is a bond;
Z is a bond or (CH₂)ₘ, wherein m = 1, 2, or 3;
Y₂ is alkylene and Y₂ is linked to Y₁ to form a C₃-C₇ cycloalkylene, preferably forming a C₄ or C₆ cycloalkylene; and
X is H.

7. The compound, the chiral enantiomer thereof, or the pharmaceutically acceptable salt thereof according to claim 2, wherein
Y₁ is CR₁, wherein R₁ is F;
Z is (CH₂)ₘ, wherein m = 1, 2, or 3;
Y₂ is alkylene and Y₂ is linked to Z to form a C₃-C₇ cycloalkylene, preferably forming a C₆ cycloalkylene; and
X is H.

8. The compound, the chiral enantiomer thereof, or the pharmaceutically acceptable salt thereof according to claim 2, wherein
Y₁ is CR₁, wherein R₁ is F;
Z is a bond or (CH₂)ₘ, wherein m = 1, 2, or 3;
Y₂ is alkylene or cycloalkylene, which is optionally substituted with alkyl, cycloalkyl, or cycloalkylene; and
X is H.

9. A compound represented by any of the following formulas, a chiral enantiomer thereof, or a pharmaceutically acceptable salt thereof:

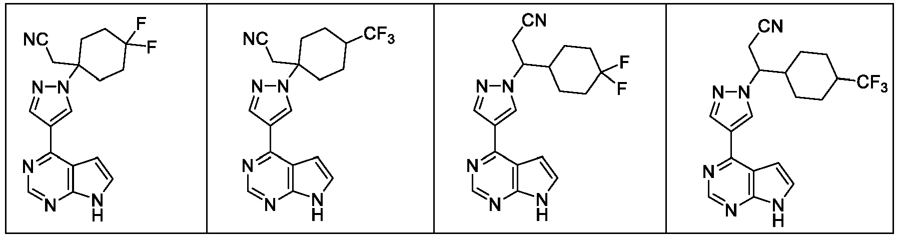

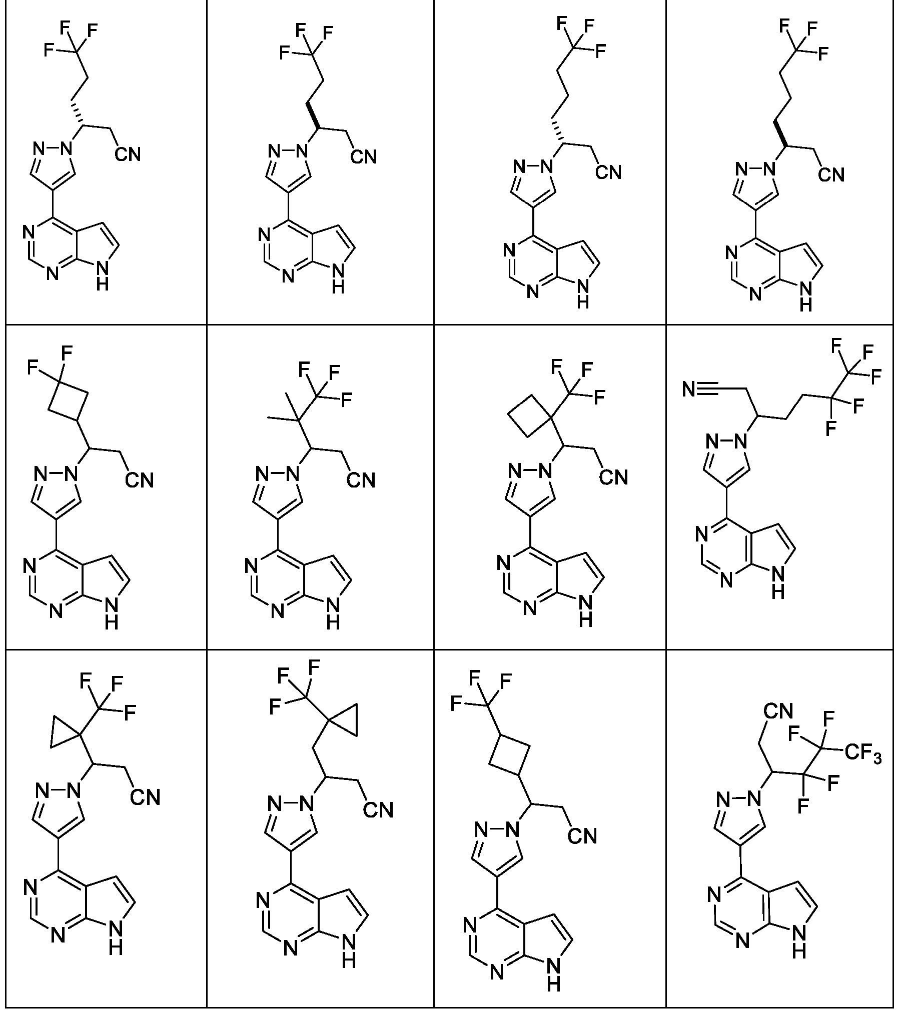

10. A method for preparing the compound, the chiral enantiomer thereof, or the pharmaceutically acceptable salt thereof according to any one of claims 1 to 9, comprising the following steps:
(1) subjecting compound III-1 and diethyl cyanomethyl phosphate to olefination reaction in the presence of alkali to produce compound III-2;
(2) subjecting the compound III-2 and compound III-3 to Michael addition reaction in the presence of alkali under heating to produce compound III; and
(3) deprotecting the compound III in the presence of alkali to produce compound I;

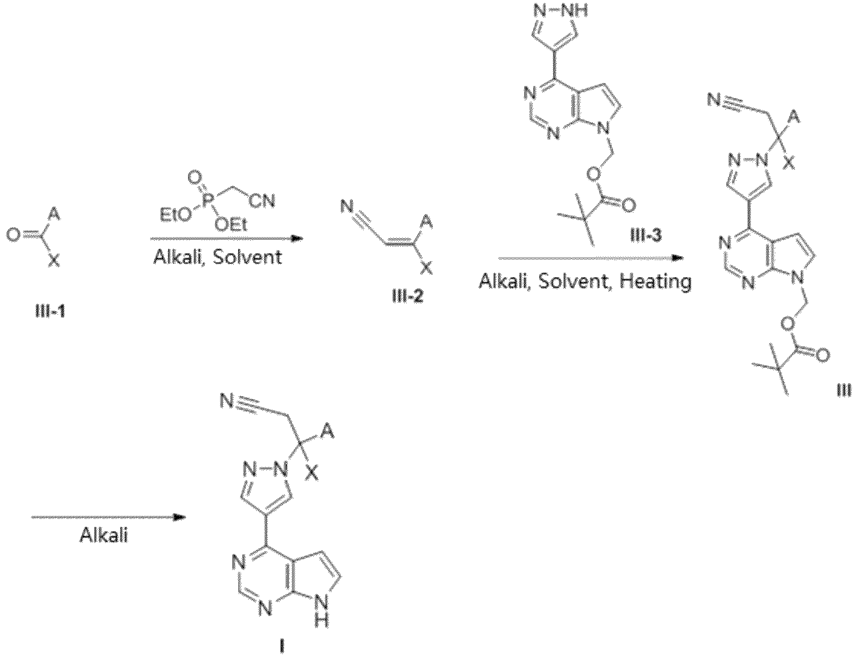

11. The method according to claim 10, wherein
the alkali used in the step (1) is selected from any one or more of sodium hydride, sodium tert-butoxide, potassium tert-butoxide, lithium tert-butoxide, lithium bromide, lithium chloride, triethylamine, 4-dimethylaminopyridine, cesium carbonate, potassium carbonate, and sodium carbonate; the solvent used in the step (1) is an aprotic solvent, and preferably, the solvent is selected from any one or more of tetrahydrofuran, dimethylformamide, and dimethyl sulfoxide; the alkali used in the step (2) is selected from any one or more of 1,8-diazabicyclo(5.4.0)undec-7-ene, sodium tert-butoxide, potassium tert-butoxide, triethylamine, 4-dimethylaminopyridine, cesium carbonate, potassium carbonate, sodium carbonate, potassium phosphate, and sodium phosphate; the solvent used in the step (2) is a protic or aprotic solvent, and preferably, the solvent is selected from any one or more of acetonitrile, N-methylpyrrolidone, dimethylformamide, dimethylsulfoxide, tetrahydrofuran, methanol, and ethanol; the heating in the step (2) is performed at a temperature of 52-82°C;
the alkali used in the step (3) is selected from any one or more of sodium hydroxide, lithium hydroxide, and potassium hydroxide.

12. An intermediate for preparing the compound, the chiral enantiomer thereof, or the pharmaceutically acceptable salt thereof according to any one of claims 1 to 9, having a structure represented by general formula (III): 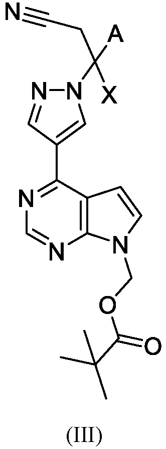 in the formula (III),
A is alkyl or cycloalkyl, wherein the alkyl or the cycloalkyl is optionally substituted with fluoro, alkyl, or cycloalkylene, and the alkyl, the cycloalkyl, or the cycloalkylene has at least one pair of gem-difluoride groups thereon;
X is H or (CH₂)ₙ, wherein n = 0, 1, 2, 3, 4, or 5; when X is (CH₂)ₙ, it is linked to A to form a C₃-C₇ cycloalkylene, wherein the cycloalkylene is optionally substituted with fluoro or alkyl, and the cycloalkylene or the alkyl has at least one pair of gem-difluoride groups thereon.

13. A method for preparing the intermediate according to claim 12, comprising the following steps:
(1) subjecting compound III-1 and diethyl cyanomethyl phosphate to olefination reaction in the presence of alkali to produce compound III-2;
(2) subjecting the compound III-2 and compound III-3 to Michael addition reaction in the presence of alkali under heating to produce compound III;

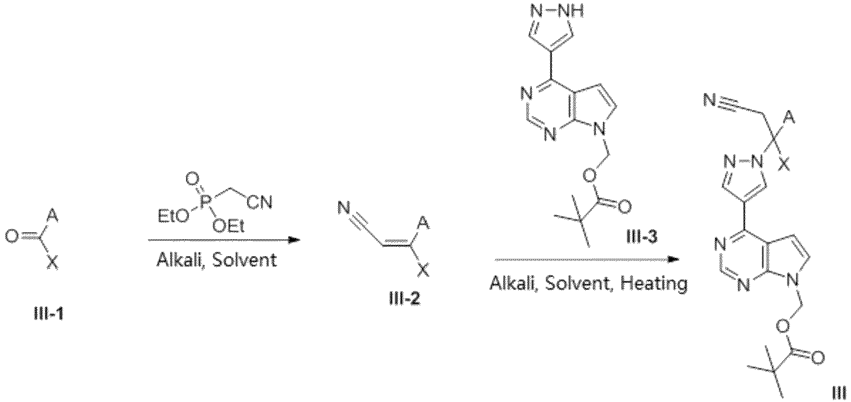

14. The method according to claim 13, wherein
the alkali used in the step (1) is selected from any one or more of sodium hydride, sodium tert-butoxide, potassium tert-butoxide, lithium tert-butoxide, lithium bromide, lithium chloride, triethylamine, 4-dimethylaminopyridine, cesium carbonate, potassium carbonate, and sodium carbonate; the solvent used in the step (1) is an aprotic solvent, and preferably, the solvent is selected from any one or more of tetrahydrofuran, dimethylformamide, and dimethyl sulfoxide; the alkali used in the step (2) is selected from any one or more of 1,8-diazabicyclo(5.4.0)undec-7-ene, sodium tert-butoxide, potassium tert-butoxide, triethylamine, 4-dimethylaminopyridine, cesium carbonate, potassium carbonate, sodium carbonate, potassium phosphate, and sodium phosphate; the solvent used in the step (2) is a protic or aprotic solvent, and preferably, the solvent is selected from any one or more of acetonitrile, N-methylpyrrolidone, dimethylformamide, dimethylsulfoxide, tetrahydrofuran, methanol, and ethanol; and the heating in the step (2) is performed at a temperature of 52-82°C.

15. A pharmaceutical composition comprising a therapeutically effective amount of the compound, the chiral enantiomer thereof, or the pharmaceutically acceptable salt thereof according to any one of claims 1 to 9, and pharmaceutically acceptable carrier, diluent, and/or excipient.

16. Use of the compound, the chiral enantiomer thereof, or the pharmaceutically acceptable salt thereof according to any one of claims 1 to 9, or the pharmaceutical composition according to claim 15 in the preparation of a medicament for the prevention and/or treatment of a disease associated with abnormal JAK signaling pathways;
preferably, the disease is an autoimmune disease, a myeloproliferative neoplastic disease, or a graft-versus-host disease;
further preferably, the autoimmune disease is selected from rheumatoid arthritis, ulcerative colitis, systemic lupus erythematosus, atopic dermatitis, or multiple sclerosis;
further preferably, the myeloproliferative neoplastic disease is selected from essential thrombocythemia, myelofibrosis, or polycythemia vera;
further preferably, the graft-versus-host disease is selected from acute graft-versus-host disease or chronic graft-versus-host disease.
